# EUROPEAN PATENT APPLICATION

(11) **EP 1 825 879 A1**
(43) Date of publication of application: **29.08.2007**
(21) Application number: 05814683.8
(22) Date of filing: 07.12.2005
(51) Int. Cl.: A61M 25/00

(54) **MEDICAL CATHETER TUBE AND PROCESS FOR PRODUCING THE SAME**

(30) Priority: 09.12.2004 JP 2004357249
(71) Applicant: Kaneka Corporation, Kita-ku Osaka-shi, Osaka 530-8288 (JP)
(72) Inventor: MURATA, Takahiro, KANEKA CORPORATION, Settsu-shi, Osaka 5660072 (JP); MIHAYASHI, Tsuyoshi, KANEKA CORPORATION, Settsu-shi, Osaka 5660072 (JP)
(74) Representative: Gille Hrabal Struck Neidlein Prop Roos
(86) International application number: PCT/JP2005/022428
(87) International publication number: WO 2006/062114

(57) **Abstract**

There is provided a medical catheter tube capable of exhibiting excellent flexibility, and provided a process for producing the same. In particular, there is provided a medical catheter tube having, arranged from a base edge side, a base part, a forefront part and a cutting edge part The medical catheter tube comprises an inner layer tube of resin pipe; a reinforcing material layer produced by knitting of a wire around the inner layer tube; a marker disposed by winding and covering of the inner layer tube at the forefront part with a roentgenopaque metal member; and an outer layer tube of resin pipe covering the reinforcing material layer and the marker. The inner layer tube, reinforcing material layer, marker and outer layer tube of the medical catheter tube are united together.

## Description

### TECHNICAL FIELD

The present invention relates to a medical catheter tube superior in flexibility, positioning efficiency, torque-transmitting efficiency, kink resistance, and pressure resistance, higher in the freedom of controlling the rigidity/flexible inclination, and allowing adjustment of rigidity/flexible balance according to various access routes, and a process for producing the same. The present invention relates to a medical catheter tube that shows favorable X-ray visibility in the distal region and also superior flexible at the same time, and a production method thereof.

### BACKGROUND ART

Catheter tubes are hollow medical devices that are used as inserted in the cavity, vessel, blood vessel, and others in the body, specifically for selective injection of angiographic agent or others, removal of blood clot, recirculation of clogged vessel, vasodilation, and the like, and are normally tube-shaped. Such a catheter demands superior usability allowing rapid, accurate, and selective insertion thereof, for example, into thin complicated-patterned blood vessel.

More specifically as for operative efficiency, such a catheter tube demands favorable positioning efficiency allowing transmission of the surgeon's operation, such as insertion and withdrawal thereof into or out of the blood vessel, from the proximal region to the distal region and favorable pressure resistance, for example, when a drug solution flows inside. It also demand favorable torque-transmitting efficiency allowing reliable transmission of the torque applied in the proximal region of catheter tube and favorable insertion efficiency allowing transmission of the surgeon's force pushing the catheter tube into the blood vessel from the proximal end to the distal end. It also demand favorable guide wire compatibility, i.e., lubricity of the internal surface of the catheter, allowing insertion and withdrawal of the catheter tube along the guide wire inserted into the blood vessel in the complicated shape smoothly without damaging the blood vessel internal wall or the like, and favorable affinity to blood and organs on the external surface of the catheter. In addition, it also demands favorable kink resistance prohibiting folding of the catheter tube in the curved or bent area of blood-vessel when the distal end of catheter tube reaches a desired position and then the guide wire is removed, and favorable distal-region flexible keeping the shape favorable for the blood vessel without damaging the blood vessel.

It is known that a structure or configuration relatively rigid in the proximal region and increasingly flexible in the direction toward the distal region is generally favorable for giving a catheter tube having properties satisfying the requirements above.

To obtain a catheter tube having the properties above, known is a method of preparing a catheter tube having an external layer formed around it, for example, by winding or braiding an internal layer tube with wire in the coli shape as a reinforcing material layer.

As the catheter tube having wire wound around an internal layer tube as a reinforcing material layer, Patent Document 1 discloses a catheter tube including a catheter main body having a region where flexible internal and external tubes are connected to each other via a reinforcing material layer, wherein the reinforcing material layer is formed by winding a strand in a lattice shape, and the catheter main body has regions higher and lower in flexural rigidity along its the axial direction because the inclination angle of the strand to the axis of the catheter main body or the distance between the strand lattice points in the axial direction of the catheter main body varies continuously or stepwise.

However, although it is possible to form a rigid proximal region and a flexible distal region on the catheter tube, the catheter tube is not aimed at raising the degree of freedom in controlling the rigidity/flexible inclination and adjusting the rigidity/flexible balance of catheter tube according to access route. In addition, there was no specific description of a marker having X-ray visibility, and thus, it is not aimed at making the catheter distal region highly flexible and ensuring X-ray visibility simultaneously.

Alternatively, as a catheter tube having wire wound around an internal layer tube as a reinforcing material layer, Patent Document 2 discloses a catheter tube having a proximal end, a distal end, and a rod-shaped tube-shaped member containing a passage as the lumen extending between these terminals, wherein the rod-shaped tube-shaped member has an internal tube-shaped liner of a first liner material coaxial with an external tube-shaped cover having a first cover material and at least one layer of a first ribbon-reinforcing material wound helically or coaxially outside the internal tube-shaped liner and covered with the external tube-shaped cover.

However, the degree of freedom in controlling the rigidity/flexible inclination is lower even in the configuration, and the elastic force of the ribbon-reinforcing material often lead to breakage of the internal tube-shaped liner or the external tube-shaped cover by the cutoff edge during production, lowering productivity. In addition, there is no concept therein to adjust the rigidity/flexible balance of the catheter tube according to access route. In addition, although an X-ray-radiopaque band is used as the X-ray visible marker, there is no description on specific embodiments of the X-ray-radiopaque band.

Yet alternatively, Patent Document 3 discloses a catheter comprising a flexible tube-shaped catheter main body and a reinforcing coil embedded in the wall of the catheter main body, wherein: the catheter main body further has a first region at the distal end of the catheter and a second region at a position closer to the proximal end from the first region; the coil extends from the first region to the second region; the coil is wound at a relatively large winding pitch over the entire length in the second region; the coil is wound at a relatively smaller winding pitch between neighboring coils over the entire length in the first region; the coil winding pitch declines gradually in the direction toward the distal end; and the rigidity of the catheter is smaller in the first region than in the second region.

However, although it is possible to form a high-rigidity proximal region and a high-flexible distal region in the catheter tube and keep a favorable balance in flexural rigidity, the rigidity/flexible balance of the catheter tube is not intended to be adjustable according to access route. Further in the catheter tube, the entire reinforcing coil is an X-ray-radiopaque metal wire; the flexible of the distal region is insufficient; and the X-ray visibility is excessively high, causing troubles in decision making by surgeons during operation.

In addition, Patent Document 4 discloses, as a catheter tube a having reinforcing material layer as braided around an internal layer tube, a vascular catheter, comprising a proximal region, a distal region, and a long shaft having a lumen extending between them, wherein: the proximal region has an internal smooth polymer layer, a reinforcement layer and external layer; respective layers have distal ends; the reinforcement layer is a braid of a metal part having multiple polymer members; and each polymer member contains multiple monofilaments.

However, although it is possible to form a rigid proximal region and a flexible distal region in the catheter tube, it is not aimed at raising the degree of freedom in controlling the rigidity/flexible inclination and adjusting the rigidity/flexible balance of catheter tube according to access route. In addition, the X-ray visible marker is a marker formed by winding a metal sheet or a metal tube around the internal layer tube, and thus, it is not possible to ensure high flexibility of the catheter distal regions in the area close to and the periphery of the marker.

Alternatively for preparing a catheter in which a fabric of strand is wound around an internal layer tube as the reinforcing material layer, Patent Document 5 discloses a method of producing a catheter comprising: forming a torque-transmitting region by covering the external surface of a thermoplastic tube containing an inserted metal core wire with a metal fabric; forming multiple insertion distal regions having a constant width at a particular gap in the tube machine direction by removing part of the fabric intermittently in the machine direction by irradiation of a laser beam at a wavelength of 1.06 µm from outside; withdrawing the metal core wire; and forming the insertion distal regions continuously in the distal regions of torque transmission regions by dividing the tube into pieces at the terminal of each insertion distal region.

However, the step of removing part of the fabric intermittently in the machine direction by irradiation of a laser beam at a wavelength of 1.06 µm is very complicated. The method is also lower in productivity, because, when a torque transmission region is formed continuously by applying a metal fabric continuously over the external surface of a thermoplastic tube having an inserted metal core wire and embedding the fabric into the outer surface of the tube by softening by heating to a depth of about 1/2 to 1/5 of the thickness in the downstream step, the metal fabric may cause problems such as ejection of the metal fabric out of the tube surface caused by the bending of cut edge due to its elastic force during embedment of the fabric by heat softening of the tube. It is also difficult to control the inclination in rigidity and flexible sufficiently. In addition, the rigidity/flexible balance of the catheter tube is not intended to be adjustable according to access route. In addition, there is no specific description on the X-ray visible marker, and thus, it is not aimed at making the catheter distal region highly flexible and ensuring X-ray visibility simultaneously.
Patent Document 1: Japanese Patent No. 3,310,031
Patent Document 2: Japanese Patent No. 2,672,714
Patent Document 3: Japanese Unexamined Patent Publication No. 2001-218,851
Patent Document 4: Japanese Unexamined Patent Publication No. 2002-535,049
Patent Document 5: Japanese Unexamined Patent Publication No. 2000-225,194

### SUMMARY OF THE INVENTION

### <Problems to be Solved by the present invention>

An object of the present invention is to provide a medical catheter tube superior in positioning efficiency, torque-transmitting efficiency, flexibility, kink resistance, pressure resistance, insertion efficiency, X-ray visibility, and others, and a process for producing the same.

In particular, it is to provide a medical catheter tube higher in the degree of freedom in controlling the rigidity/flexible inclination and allowing adjustment of the rigidity/flexible balance according to access route simultaneously, because the medical catheter tube according to the present invention is used in various affected areas and the access route to the affected area may vary significantly, and to disclose a process for producing the same.

Another object of the present invention is to provide a medical catheter tube having a marker of an X-ray-radiopaque metal placed in the distal region side of reinforcing material layer that is flexible to bending deformation and shows favorable X-ray visibility and high flexibility of the distal region at the same time, and a process of producing the same.

### <Means to Solve the Problems>

Accordingly, the present invention (1) relates to a medical catheter tube having a proximal region, a distal region and a most distal region from the proximal end, comprising: an internal layer tube of a resin tube; a reinforcing material layer of a wire braided on the internal layer tube; a marker formed by winding an X-ray-radiopaque metal part around the internal layer tube in the distal region; and an external layer tube of resin tubes covering the reinforcing material layer and the marker, wherein: the internal layer tube, the reinforcing material layer, the marker and the external layer tube are integrated; the wire for the reinforcing material layer includes a synthetic resin wire and/or a metal wire; the reinforcing material layer is formed only in the proximal region; the marker is flexible to bending deformation; and the flexural rigidity of the external layer tube decreases stepwise or continuously in the direction from the proximal region to the distal region.

The present invention (2) also relates to the catheter tube according to (1), wherein: the resin tube is lubricating and flexible; the wire for the reinforcing material layer provides the catheter tube with kink resistance, pressure resistance, torque-transmitting efficiency, insertion efficiency, and others; and the external layer tube is flexible.

The present invention (3) also relates to the medical catheter tube according to (1) or (2), wherein the medical catheter is a tube formed with an X-ray-radiopaque metal wire coil wound around the internal layer tube, a square X-ray-radiopaque metal sheet wound around the internal layer tube that has slits cut from both sides, or a tube of a resin containing a kneaded X-ray-radiopaque metal powder.

The present invention (4) also relates to the medical catheter tube according to any one of (1) to (3), wherein the wire for the reinforcing material layer is a synthetic fiber having a thermoplastic liquid crystal polymer as its internal core and a flexible polymer as its sheath.

The present invention (5) also relates to the medical catheter tube according to any one of (1) to (4), wherein the pick distance of the braid for the reinforcing material layer changes continuously or stepwise in the direction from the proximal region to the distal region.

The present invention (6) also relates to the medical catheter tube according to any one of (1) to (5), wherein the external layer tube has multiple segments, which are aligned in such a manner that the Shore D hardness of the resins for the segments decreases stepwise in the direction from the proximal region to the distal region.

The present invention (7) also relates to the medical catheter tube according to any one of (1) to (6), wherein the external diameter of the external layer tube is altered and the catheter tube is formed in the rounded or tapered shape in the most distal region.

The present invention (8) also relates to the medical catheter tube according to any one of (1) to (7), wherein the external layer tube is coated hydrophilically.

The present invention (9) also relates to a process of producing the medical catheter tube according to the present invention, comprising forming a reinforcing material layer on the external surface of an internal layer tube by braiding, forming an X-ray-radiopaque marker flexible to bending deformation to the distal side of the reinforcing material layer, and coating an external layer tube, wherein the X-ray-radiopaque marker is formed by winding an X-ray- radiopaque metal wire in the coil shape or by placing a square X-ray-radiopaque metal sheet having slits from the both sides around the internal layer tube to the distal side of the reinforcing material layer or by using a resin containing a kneaded X-ray-radiopaque metal powder and thus the catheter tube has a flexible distal region.

The present invention (10) relates to a process of producing the medical catheter tube according to the present invention, comprising forming a reinforcing material layer on the external surface of an internal layer tube by braiding, forming an X-ray-radiopaque marker flexible to bending deformation to the distal side of the reinforcing material layer, and coating an external layer tube, wherein the reinforcing material layer is formed by braiding a wire supplied from a wire supplying unit on the external surface of the internal layer tube in such a manner that the braid pick distance changes continuously or stepwise by changing the relative traveling speeds of the internal layer tube and the wire fed from the supplying unit.

The present invention (11) also relates to a process of producing the medical catheter tube according to the present invention, comprising forming a reinforcing material layer on the external surface of an internal layer tube by braiding, forming an X-ray-radiopaque marker flexible to bending deformation to the distal side of the reinforcing material layer; and coating an external layer tube, wherein: one or more resin tubes different in Shore D hardness are formed as the external layer tube; and the rigidity/flexible balance of the catheter tube can be altered in various ways by aligning the resin tubes for external layer tube in such a manner that the Shore D hardnesses thereof decreases in the direction from the proximal region to the distal region and also in such a manner that the braid pick distance of the reinforcing material layer changes continuously or stepwise when multiple resin tubes different in Shore D hardness are formed.

The present invention (12) also relates to a process of producing the medical catheter tube according to the present invention, comprising forming a reinforcing material layer on the external surface of an internal layer tube by braiding, forming an X-ray-radiopaque marker flexible to bending deformation to the distal side of the reinforcing material layer, and coating an external layer tube, further comprising: forming one or more resin tubes different in Shore D hardness as the external layer tube; aligning the resin tubes for external layer tube in such a manner that the Shore D hardnesses thereof decreases stepwise in the direction from the proximal region to the distal region for making the stepwise change of the resin tubes different in Shore D hardness, covering the entire composite with a shrink tube, integrating the internal layer tube, the reinforcing material layer, the X-ray-radiopaque marker, and the external layer tube by heating the composite, converting the most distal region thereof into the rounded or tapered shape, and removing the shrink tube after cooling.

The present invention (13) relates to a process of producing the medical catheter tube according to the present invention, comprising forming a reinforcing material layer on the external surface of an internal layer tube by braiding, forming an X-ray-radiopaque marker flexible to bending deformation to the distal side of the reinforcing material layer, and coating an external layer tube, further comprising forming one or more resin tubes different in Shore D hardness as the external layer tube, by coating extrusion, on a structure having a reinforcing material layer formed by coating extrusion molding on the external surface of the internal layer tube in such a manner that the Shore D hardness changes stepwise, forming the resin tubes for external layer tube by coating extrusion in such a manner that the Shore D hardnesses thereof decreases stepwise in the direction from the proximal region to the distal region for making the stepwise change of the resin tubes different in Shore D hardness, integrating the internal layer tube, the reinforcing material layer, the X-ray-radiopaque marker, and the external layer tube, and converting the most distal region into the rounded or tapered shape.

### <Advantageous Effects of the Invention>

By the means to solve problems according to the present invention (1) described above, the present invention provides a medical catheter tube superior in positioning efficiency allowing movement in accordance with guide wire and torque-transmitting efficiency when a surgeon applied a rotational force that has flexibility changing in the direction from the proximal region to the distal region. The configuration is also effective in providing a medical catheter tube giving a high degree of freedom in adjusting the rigidity/flexible balance and allowing adjustment of the rigidity/flexible balance according to access route, that shows kink resistance causing no crimping when bent in a complicated way and is superior in pressure resistance, guide wire compatibility, productivity, and others. The present invention gives a catheter tube showing favorable X-ray visibility, by forming a marker thereon by winding an X-ray-radiopaque metal part around the internal layer tube in the distal region, and, when the marker is flexible enough to bending deformation, the catheter becomes highly flexible in the particularly important distal and most distal regions. Thus, the present invention gives a medical catheter tube highly flexible in the distal region.

According to the present invention (2), when the resin tube is lubricating and flexible, the wire provides the catheter tube with kink resistance, pressure resistance, torque-transmitting efficiency, insertion efficiency, and others, and the external layer tube is flexible, it is possible to move the catheter tube smoothly forward and backward when a guide wire is inserted into the resin tubes of the catheter tube, and thus, to send the catheter tube together with both the wire and the external layer tube to a desirable treatment area.

According to the present invention (3), because the marker is an X-ray-radiopaque metal wire coil wound around the internal layer tube, a square X-ray-radiopaque metal sheet having slits cut from both sides wound around the internal layer tube, or a tube of a resin kneaded with a X-ray-radiopaque metal powder, it is possible to obtain a soft catheter tube that does not become hardened in the distal region.

According to the present invention (4), because the wire has a thermoplastic liquid crystal polymer as its internal core and a flexible polymer as its sheath, the catheter tube is improved in reinforcement effects such as pressure resistance and kink resistance, and the marker of an X-ray-radiopaque metal part in the distal region becomes more visible during use under X-Ray irradiation.

According to the present invention (5), advantageously because the pick distance of the braid forming the reinforcing material layer changes continuously or stepwise in the direction from the proximal region to the distal region, the rigidity of the catheter tube decreases gradually in the direction from the proximal region to the distal region.

According to the present invention (6), advantageously because the external layer tube has multiple segments and the multiple segments are aligned in such a manner that the Shore D hardness of the resins for the segments decreases stepwise in the direction from the proximal region to the distal region, the hardness of the catheter tube decreases gradually in the direction from the proximal region to the distal end and also the torque applied to the proximal region is transmitted more easily to the distal region.

According to the present invention (7), favorably because the external layer tube is modified in its external diameter and molded in the rounded or tapered shape in the most distal region, the catheter tube does not damage the internal wall of blood vessel during use.

According to the present invention (8), because the external layer tube is coated hydrophilically, such a medical catheter tube can be inserted into the lumen of the body easily during use.

The present inventions (9) to (13) relates to the process of producing the medical catheter tube according to the present invention.

### BRIEF DESCRIPTION OF THE DRAWINGS

Figure 1 is a flowchart showing a production process of the present invention.
Figure 2 is a schematic view illustrating a metal core wire wound around a reel.
Figure 3 is a schematic explanatory drawing illustrating a method of forming internal layer tube continuously by coating in an extruder.
Figure 4 is a schematic explanatory drawing illustrating a method of braiding wire around an internal layer tube and thus forming a reinforcing material layer.
Figure 5 is an expanded side view illustrating picks and the pick distance.
Figure 6 is an expanded side view illustrating a wire placed in the axial direction as a reinforcing material.
Figure 7 is a crosssectional view illustrating the structure of a wire favorably used as a synthetic resin wire; Figure (a), an expanded perspective view of the wire terminal; and Figure (b), a scanning micrograph of the wire terminal.
Figure 8 is a schematic explanatory view illustrating a catheter tube from which the internal layer tube and the reinforcement layer are removed at the positions corresponding to the catheter distal region and the proximal region.
Figure 9 is a schematic side view illustrating a single catheter after cleavage.
Figure 10 is a schematic side view illustrating a catheter having an X-ray-radiopaque marker placed at catheter distal end.
Figure 11 is a side view illustrating a square X-ray-radiopaque metal sheet marker having slits from both sides.
Figure 12 is a partial schematic side view illustrating a catheter having a square X-ray-radiopaque metal sheet marker having slits from both sides placed at the catheter distal end.
Figure 13 is an expanded side view illustrating a catheter having an X-ray-radiopaque metal wire marker with its internal layer tube and reinforcement layer removed at the positions corresponding to the catheter distal region and the proximal region.
Figure 14 is an expanded side view illustrating a catheter having an X-ray-radiopaque metal wire marker with its internal layer tube and reinforcement layer removed at the positions corresponding to the catheter distal region and the proximal region.
Figure 15 is a schematic crosssectional side view illustrating a catheter having four resin tubes different in Shore D hardness placed in contact with each other as an external layer.
Figure 16 is a schematic crosssectional side view illustrating a catheter having an external layer tube changing stepwise in Shore D hardness.
Figure 17 is a schematic sectional view illustrating a catheter having a formed shrink tube.
Figure 18 is a schematic sectional view illustrating a catheter having an internal layer tube, a reinforcing material layer, and an external layer tube integrated by shrinkage of a shrink tube wherein the resin tube distal region of the external layer tube is molded into the rounded shape.
Figure 19 is a schematic sectional view illustrating the distal end of a composite tube and a heat mold for molding the distal region.
Figure 20 is a schematic sectional view illustrating the distal end of a composite tube in contact with the distal region-forming mold under heat.
Figure 21 is a schematic sectional view illustrating a catheter after the shrink tube is removed.
Figure 22 is a schematic explanatory view illustrating the catheters wound around a reel as a continuous unit while the metal core wires are welded.
Figure 23 is a schematic explanatory view illustrating the process of an external layer being formed by coating extrusion.
Figure 24 is a schematic sectional view illustrating a single catheter after cleavage having a shrink tube placed at the distal end.
Figure 25 is a schematic sectional view illustrating the catheter after the metal core wire is withdrawn and the proximal end cross section is finished.
Figure 26 is a conceptual diagram showing the rigidity/flexible balance.

### EXPLANATION OF REFERENCES

1: Metal core wire
2: Reel
3: Internal layer tube
4: Extruder
5: Reinforcing material layer
6: Core of thermoplastic liquid crystal polymer
7: Island (sheath) of thermoplastic liquid crystal polymer
8; Sea (sheath) of flexible polymer
9: Metal core wire
10: X-ray-radiopaque metal wire marker
11: Square X-ray-radiopaque metal sheet marker having slits from both sides
12: Square X-ray-radiopaque metal sheet marker having slits from both sides wound
13: Metal core wire
14: X-ray-radiopaque metal wire marker
15: X-ray-radiopaque metal sheet marker wound having slits from both sides
16: External layer tube
16a: Maximum-Shore D hardness external layer tube
16b: High-Shore D hardness external layer tube
16c: Low-Shore D hardness external layer tube
16d: Minimum-Shore D hardness external layer tube
17: X-ray-radiopaque marker
18: Shrink tube
19: Rounded molded part
20: Heat mold
21: Heat-molded tapered distal region
22: Spot welding machine
23: Extrusion mold
24: Extruder
25: Shrink tube
P: Pick
a: Pick distance

### BEST MODE OF CARRYING OUT THE INVENTION

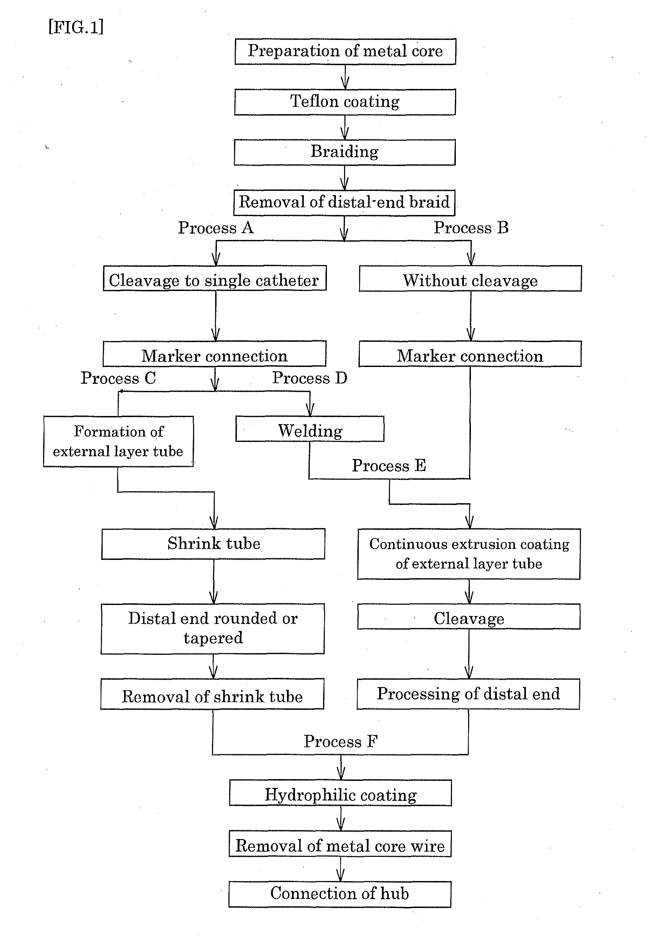

Hereinafter, the best mode of the medical catheter tube according to the present invention will be described with reference to drawings. These drawings are schematic drawings showing the characteristics of the configuration of the present invention, and the length and diameter of each region is arbitrary, if the catheter tube can be used favorably as a catheter tube for medical treatment. Figure 1 is a flowchart showing the production process, and the best mode of the present invention will be described with reference to the Figure. In the present invention, various modifications are possible within the scope of the present invention specified by its claims.

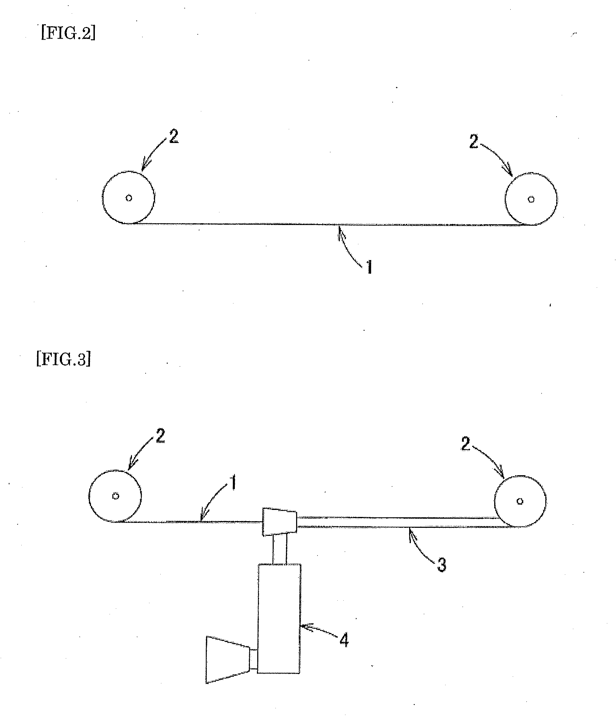

First, a metal core wire 1 is made available, as shown in Figure 2. The metal core wire 1 is wound around reels 2; the external diameter of the wire corresponds roughly to the internal diameter of the catheter to be produced; and it is preferably a metal-plated conductor or a stainless steel wire. For convenience, in all Figures 2 and below, the left side of the wire represents the proximal region, while the right side, the distal region.

As shown in Figure 3, an internal layer tube 3 is formed on the metal core wire 1 with an extruder 4 by extrusion coating. The material for the internal layer tube 3 is not particularly limited if it is a resin, and examples thereof include fluorine resins such as polytetrafluoroethylene, tetrafluoroethylene-perfluoroalkyl vinylether copolymers, tetrafluoroethylene-hexafluoropropylene copolymers, and ethylene-tetrafluoroethylene copolymers; polyolefins such as polypropylene, polyethylene, and ethylene-vinyl acetate copolymers; polyamides; polyesters such as polyethylene terephthalate and polybutylene terephthalate; polyurethane, polyvinyl chloride, polystyrene resins, polyimide, and other resins, and the mixture thereof, but favorable for making the final product more lubricant, for example, to the guide wire extending in the internal layer tube and have favorable positioning efficiency and guide wire compatibility, i.e., from the viewpoint of flexibility, are fluorine resins such as polytetrafluoroethylene and tetrafluoroethylene-perfluoroalkyl vinylether copolymers.

The internal layer tube 3 that coats the metal core wire 1 preferably has sufficiently high adhesiveness to the metal core wire 1. For the purpose of increasing the adhesiveness between the internal layer tube 3 and the external layer tube, the surface of the internal layer tube may be roughened or modified by a mechanical method (for example, abrasion of internal layer tube surface with a sand paper), a chemical method (use of a defluorinating agent such as sodium naphthalene in dimethylether), or an electrical method such as plasma processing, in the subsequent step of coating an external layer tube.

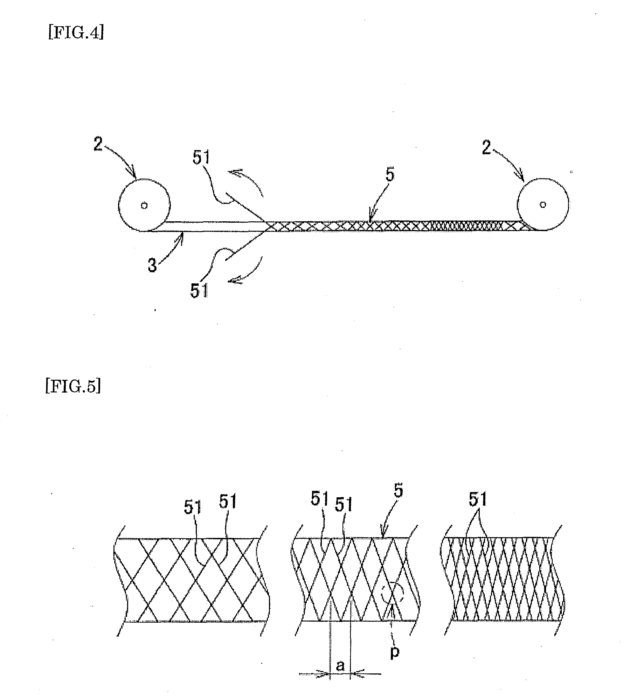

There are many kinds of braiding patterns such as one-over/one-under and two-over/two-under, but any one of them may be used if it is suitable for a reinforcing material layer 5 for catheter.
Then, a reinforcing material layer 5 is formed by braiding a wire 51 around the internal layer tube 3, as shown in Figure 4. The wire 51 provides the catheter tube with kink resistance, pressure resistance, torque-transmitting efficiency, insertion efficiency, and others. The wire is braided in a braiding machine. Each of the stitches is called a pick, and as shown in Figure 4, it becomes possible to adjust the rigidity/flexible inclination or the rigidity/flexible balance described below, by making the pick distance change continuously or stepwise, as it is narrower in the distal region and wider in the proximal region of catheter. Figure 5 is an expanded schematic view of the braid, and the intersection (p) in the Figure is called a pick, and the stitch distance (a), a pick distance.

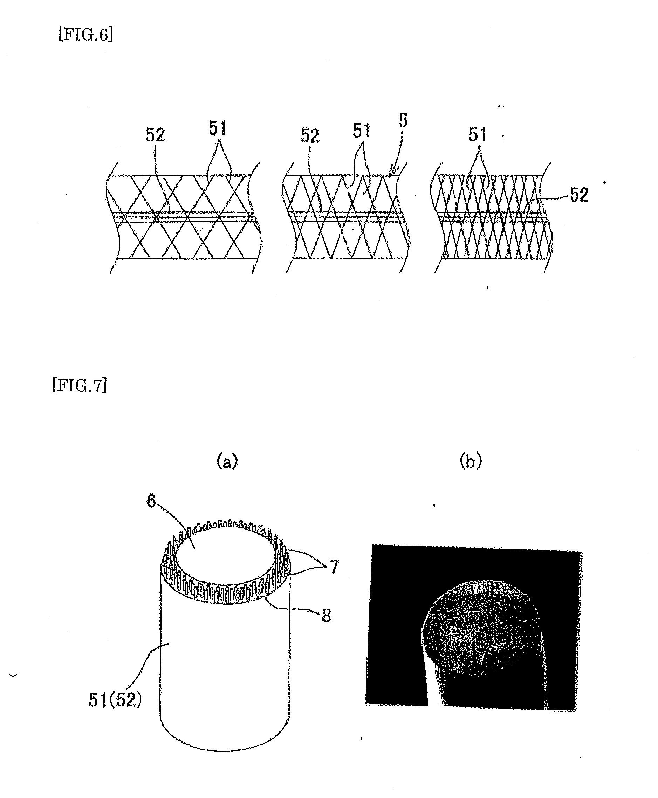

A smaller pick distance leads to increase in flexibility, while a larger pick distance to increase in rigidity. The filament number and the stitch number of the braid are selected arbitrarily. The filament number is the number of the wires 51 contained in one pick, and the stitch number is the number of picks per unit length.
As shown in Figure 6, a suitable number of wires 52 may be placed in the axial direction of the reinforcing material layer 5 for control of elongation of the catheter when pulled or to make the catheter more compatible with the curvature in complicated blood vessel. Presence of the wires 52 in the axial direction may lead to smoothening of the rigidity/flexible inclination and increase of the bursting resistance.

A synthetic resin wire may be used together with a metal wire as the wires 51 and 52. Particularly favorably used as the synthetic resin wire is a wire having a core 6 of a thermoplastic liquid crystal polymer and a coating layer containing islands of a thermoplastic liquid crystal polymer (sheath) 7 and a sea of a flexible polymer (sheath) 8, as shown in the schematic crosssectional view and the scanning micrograph shown in Figure 7. The thermoplastic liquid crystal polymer is for example polyarylate, and the flexible polymer, polyethylene naphthalate. The diameter of the synthetic resin wire favorably used is preferably 5 to 50 µm. Examples of the wires 51 and 52 are disclosed in Japanese Unexamined Patent Publication No. 2002-20,932.

Examples of the other synthetic resin for the wire include polyesters such as polyethylene terephthalate, polybutylene terephthalate, and polymethylene terephthalate; polyolefins such as polyethylene and polypropylene, hard polyvinyl chloride, polyamide, polyimide, polystyrene, thermoplastic polyurethane, polycarbonate, ABS resins, acrylic resins, polymethyl methacrylate, polyacetal, polyarylates, polyoxymethylene, high-tension polyvinylalcohol, fluoroplastics, polyvinylidene fluoride, polytetrafluoroethylene, ethylene-vinyl acetate hydrolysates, polysulfone, polyether sulfone, polyether ketone, polyphenyleneoxide, polyphenylene sulfide, aromatic polyaramides such as Kevlar (registered trade name of E.I. du Pont de Nemours and Company in U.S.), polymer alloys containing at least one of the resins above, carbon fiber, and glass fiber.

Examples of the metal wires include those of various metals such as stainless steel, copper, tungsten, nickel, titanium, music wire, superelastic alloys such as Ni-Ti alloy, Ni-Ti-Co alloy, Ni-Al alloy, Cu-Zn alloy, and Cu-Zn-X alloy (for example, X is Be, Si, Sn, Al, or Ga), and amorphous alloys, and, among these materials, use of stainless steel is preferable for making the visibility thereof lower than that of X-ray-radiopaque marker for ensuring visibility of the X-ray-radiopaque marker later placed and also for assuring better processability, cost, and safety. The metal wire preferably has a diameter of approximately 5 to 50 µm.

The synthetic resin wire and the metal wire may be used as a single wire or as an aggregate (e.g., twisted or bundled wires). In the present invention, only a synthetic resin wire or a metal wire may be used, or both a synthetic resin wire and a metal wire are used in combination.

After the reinforcing material layer 5 is formed, a bonding layer not shown in the Figure may be formed additionally to fix it to the internal layer tube 3. The bonding layer, which is aimed at blocking small pores generated in the internal layer tube 3 and increasing anti-bursting strength, is formed by coating or spray-coating a soft polyurethane, a polyurethane dispersion or a soft adhesive agent on the internal layer tube 3 and the braided reinforcing material layer 5 to a thickness of 5 to 50 µm.

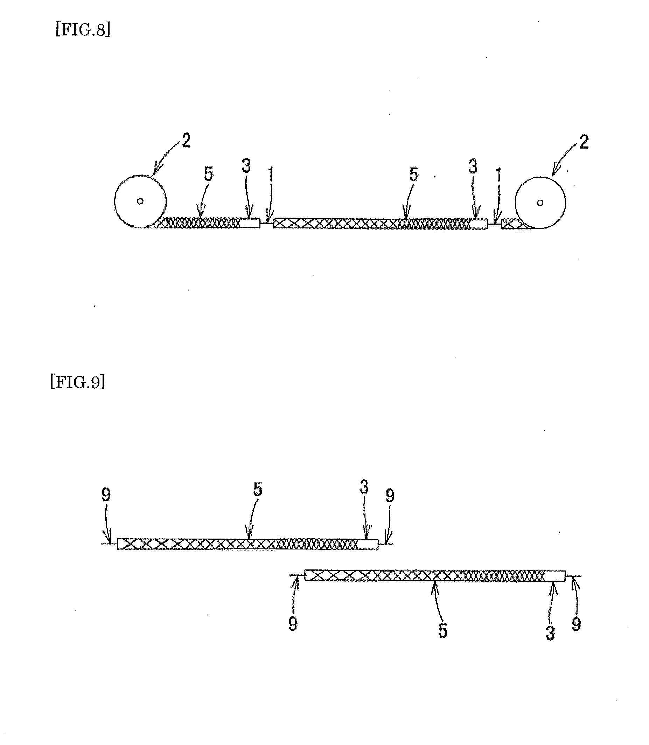

Subsequently as shown in Figure 8, the internal layer tube 3 and the reinforcing material layer 5 at the positions corresponding to the distal and proximal regions of the catheter are removed, leaving the metal core wire 1 exposed. The polyurethane, polyurethane dispersion or soft adhesive agent may be coated then on the braid distal and proximal ends, for fixing the braid to the internal layer tube 3.

Subsequently as show in the flowchart of Figure 1, a process A or B may be carried out. The marker is formed by winding an X-ray-radiopaque metal part around the distal region of the internal layer tube 3 and may be any material, if it is flexible to bending deformation. It is possible to determine whether the marker is "flexible to bending deformation" or not by comparing the flexural rigidity of the X-ray-radiopaque metal part with the flexural rigidity of a simple X-ray-radiopaque metal tube.

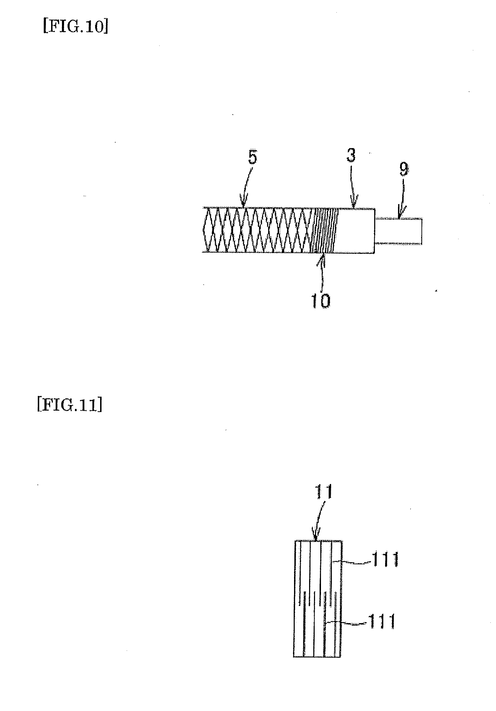

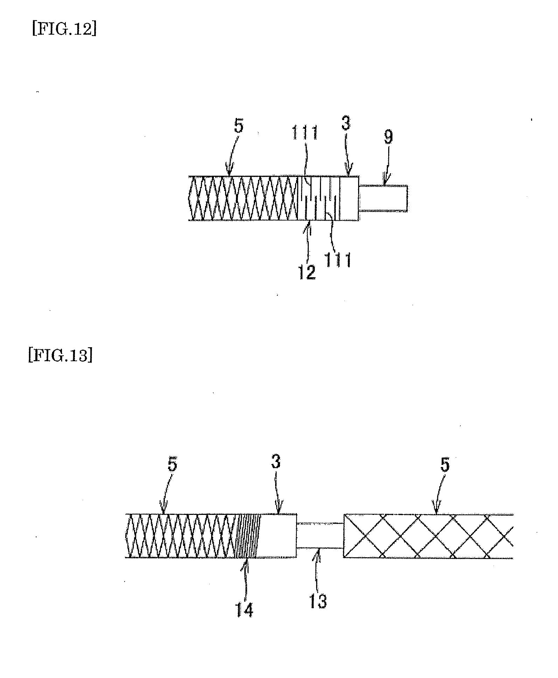

The process A will be described first.
In the process A, a catheter is formed by cutting the exposed metal core wire 1, as shown in Figure 9. The catheter tube cut has at least a proximal region, a distal region, and a most distal region from the proximal end. Figure 10 is an expanded view of the catheter distal region, while Figure 9 is a view illustrating the metal core wire after cutoff. An X-ray-radiopaque metal wire marker 10 is placed densely at the distal end of the reinforcing material layer 5, and, for that purpose, an X-ray-radiopaque metal wire is wound as a coil around the internal layer tube 3 in the distal region of the catheter tube. The X-ray-radiopaque metal wire may be wound around the internal layer tube 3 densely with the metal wires in contact with each other or coarsely with the metal wires separated from each other. An X-ray-radiopaque metal sheet marker 11 with slits 111 from the sides in the shape shown in Figure 11 is placed as wound around the internal layer tube 3 to the distal region of the reinforcing material layer 5 as shown in Figure 12. Figure 12 is an expanded view of the catheter distal region, in which code 12 represents an X-ray-radiopaque metal sheet marker 11 that is wound around the internal layer tube 3.

The diameter of the X-ray-radiopaque marker is preferably 5 to 50 µm when a metal wire is used, and the thickness thereof is preferably 5 to 30 µm when a metal sheet is used. The X-ray-radiopaque marker shows favorable flexibility, when a metal wire or a metal sheet is used. The X-ray-radiopaque marker 10 or 12 may be fixed on the internal layer tube 3 as needed, for example, by using an adhesive agent. A metal higher in X-ray impermeability and X-ray visibility such as platinum (Pt), a Pt-Ir alloy, a Pt-W alloy, a Pt-Ni alloy, gold, or silver is used favorably as the material for the X-ray-radiopaque markers 10 and 12.

Alternatively, although not shown in the Figure, a resin tube containing a kneaded X-ray-radiopaque metal powder such as barium sulfate, bismuth oxide, bismuth subcarbonate, bismuth tungstate, or bismuth-oxychloride may be formed to the distal side of the reinforcing material layer 5 on the internal layer tube 3. The resin for use is preferably the same as that for the external layer tube described below. The resin tube containing a kneaded X-ray-radiopaque metal powder may be placed then, as it is in the original tube shape or cut in the axial direction. As will be described below, the distal region of the external layer tube may also be formed with a resin containing a kneaded X-ray-radiopaque metal powder. These processings are performed in the process A.

The process B is a process of placing an X-ray-radiopaque marker without cleavage of the catheter.

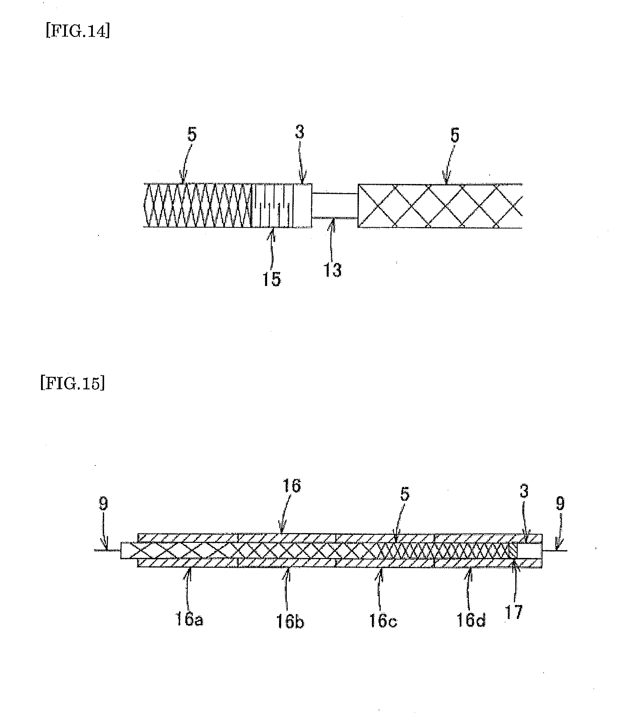

Figure 13 is an expanded view illustrating the catheter proximal region shown in Figure 8. 13 represents a metal core wire, and an X-ray-radiopaque metal wire marker 14 is placed to the distal side of the reinforcing material layer 5 as it is wound around the internal layer tube 3, similarly to the metal core wire in Figure 10. The X-ray-radiopaque metal wire may be wound around the internal layer tube 3 densely with the metal wires in contact with each other or coarsely with the metal wires separated from each other. Alternatively, an X-ray-radiopaque metal sheet marker 15 with slits from the sides in the shape shown in Figure 11 is placed as wound around the internal layer tube 3 to the distal region of the reinforcing material layer 5 as shown in Figure 14. The shape and the material for the X-ray-radiopaque marker are the same as those described above. Alternatively, although not shown in the Figure, a resin tube containing a kneaded X-ray-radiopaque metal powder may be formed to the distal side of the reinforcing material layer around the internal layer tube, similarly to above.
These processings are performed in the process B.

The process C is a step of fixing an external layer tube 16 on the catheter prepared in process A. The flexural rigidity of the external layer tube 16 should decrease stepwise or continuously in the direction from the proximal region to the distal region. The degree of the flexural rigidity in the present description corresponds to the value of Shore D hardness of the resin for the external layer tube 16.

The external layer tube 16 is preferably flexible, for the purpose of sending the catheter tube through blood vessel to the treatment site, transmitting the torque of the proximal region to the distal region, and making the marker placed by winding an X-ray-radiopaque metal part around the internal layer tube 3 in the distal region show flexibility. The external layer tube 16 is formed in such a manner that resin tubes 16a to 16d for the external layer tube 16 have Shore D hardnesses increasing from the proximal region to the distal region, as shown in Figure 15. In the distal region, the resin tube is formed as it extends to the distal end beyond the X-ray-radiopaque marker 17. Preferably, the external layer resin tube 16 has multiple segments, and the multiple segments are aligned in such a manner that the Shore D hardnesses of the resins for the segments decrease stepwise in the direction from the proximal region to the distal region. Four kinds of segments different in Shore D hardness are placed densely in contact with each other in Figure 15, in such a manner that the Shore D hardness decreases gradually in the direction from the proximal region to the distal region.

The Shore D hardness in the present description is a value determined by a type-D durometer according to ISO 7619.
Thus, the Shore D hardness of the resin tubes for the external layer tube 16 is in the order of 16a > 16b > 16c > 16d in Figure 15. Resins having a Shore D hardness of approximately 20 to 80 are used favorably. When an external layer tube 16 made of a single resin having a particular Shore D hardness is placed, the external layer tube 16 having the single kind of Shore D hardness may be cut into pieces and then placed thereon. Preferably, there is a very thin gap between the composite of the internal layer tube 3 and the reinforcing material layer 5 braided thereon and the resin tube of external layer tube 16, and in such a configuration, the wire for the reinforcing material layer 5 is less disturbed. The resin tubes for the external layer tube 16 different in Shore D hardness allows gradual change of the catheter tube in the rigidity/flexible inclination, when placed at positions separated from the position where the pick distance of braid is changing.

Although not shown in the Figure, when a marker is formed with a resin containing a kneaded X-ray-radiopaque metal powder without use of an X-ray-radiopaque metal wire marker or an X-ray-radiopaque metal sheet marker, a short resin tube may be placed at the most distal region of the resin tube for the external layer tube.

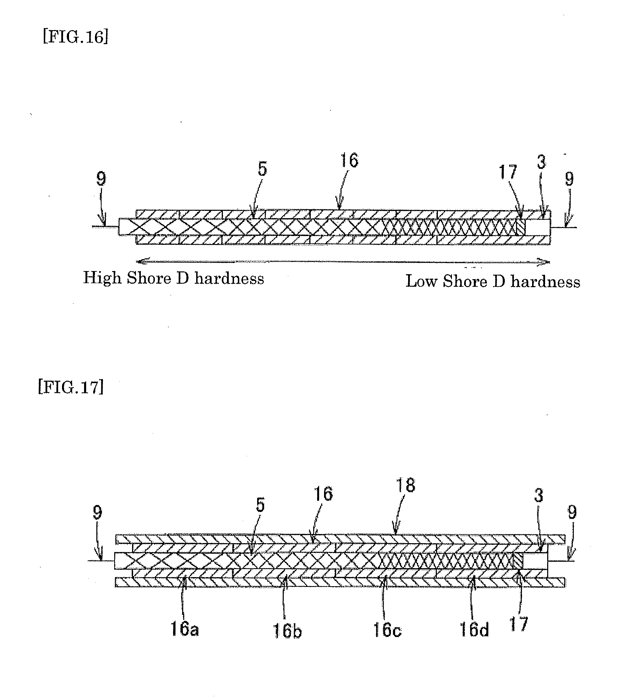

Alternatively, the resin tube for the external layer tube 16 may be prepared by preparing a resin tube having a Shore D hardness changing stepwise by using plural extruders connected to an extrusion mold and feeding the resins different in Shore D hardness from the plural extruders one by one by operation and termination, and placing the resin tube around the internal layer tube 3 having a braided reinforcing material layer 5, as shown in Figure 16. Yet alternatively, the resin tube may be prepared by preparing a resin tube having a Shore D hardness changing stepwise by connecting plural extruders to a mold having a valve mechanism, continuously extruding resins different in Shore D hardness into the extrusion channel while extrusion and discharge are switched, and placing the resin tube around the internal layer tube 3 having a braided reinforcing material layer 5, as shown in Figure 16. Then, the external layer tubes 16 should be so placed that it has a higher Shore D hardness in the region closer to the proximal terminal and a lower Shore D hardness in the region closer to the distal end. Although not shown in the Figure, the most distal region of the resin tube may be formed with a resin containing a kneaded X-ray-radiopaque metal powder in these methods.

Examples of the materials for the resin tubes of the external layer tube 16 include various elastomers such as polyamide elastomer, polyester elastomer, polyurethane-elastomer, polystyrene elastomer, fluorine-based elastomer, silicone rubber, and latex rubber, and two or more of them may be used in combination.

The polyamide elastomer is a concept including block copolymers having a hard segment of an aliphatic or aromatic polyamide such as of nylon 6, nylon 64, nylon 66, nylon 610, nylon 612, nylon 46, nylon 9, nylon 11, nylon 12, an N-alkoxymethyl-modified nylon, a hexamethylenediamine-isophthalic acid condensate polymer, or a meta-xyloyldiamine-adipic acid condensate polymer and a soft segment of a polymer such as polyester or polyether; polymer alloys of the polyamide above and a high-flexibility resin (polymer blend, graft polymerization, random polymerization, etc.); the polyamides above softened for example with a plasticizer; and the mixtures thereof.

The polyester elastomer is a concept including block copolymers of a saturated polyester such as polyethylene terephthalate or polybutylene terephthalate with polyether or polyester, and additionally, the polymer alloys thereof, the saturated polyesters softened for example with a plasticizer, and the mixture thereof.

The material favorably used is preferably a polyamide elastomer from the viewpoints of its processability and flexibility, and a typical example thereof is PEBAX manufactured by ElfAtochem.

Then, as shown in Figure 17, a shrink tube 18 that shrinks in its diameter under heat is placed on the entire external surface of the external layer tube 16. The material for the shrink tube 18 is preferably polytetrafluoroethylene, a perfluoroethylene-propene copolymer, or the like.

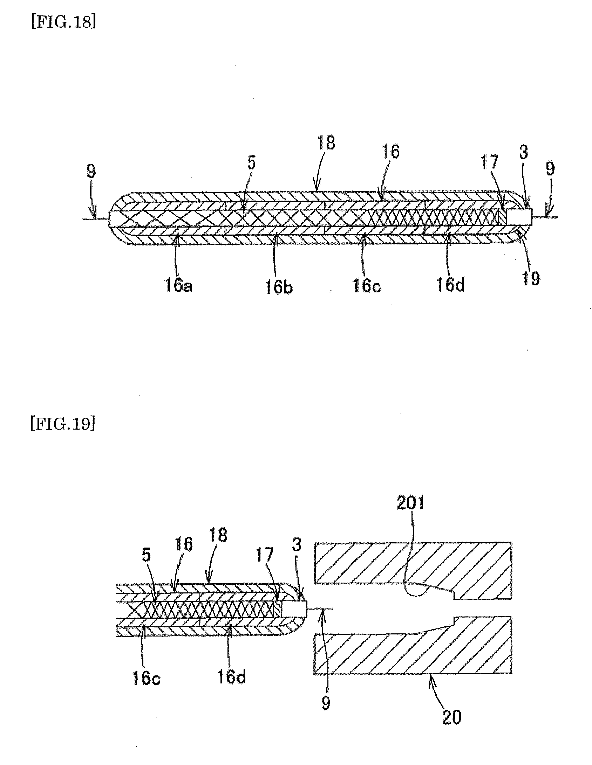

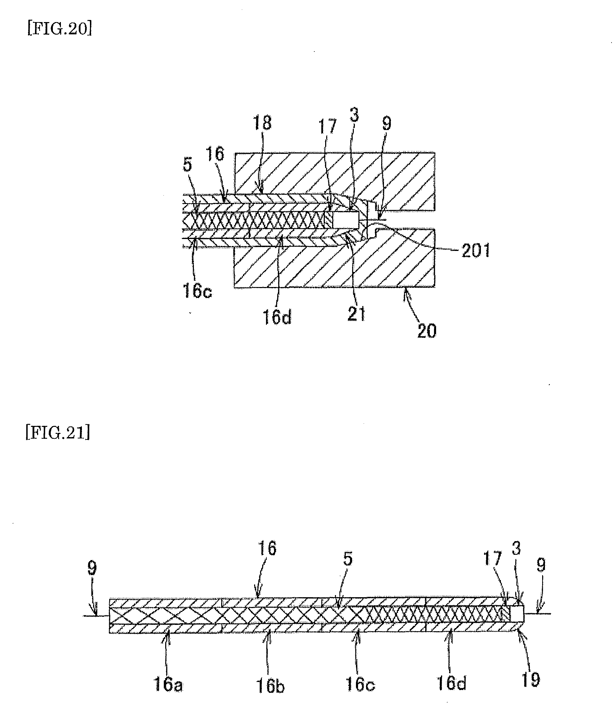

Then, the internal layer tube 3, the reinforcing material layer 5, and the external layer tube 16 are integrated, while the shrink tube 18 is heated to a tube-contracting temperature by a heater or application of high-frequency electromagnetic wave. Contraction of the shrink tube 18 results in converting of the distal region of the external layer tube 16 into a rounded shape 19, as shown in Figure 18. In converting the distal region of the external layer tube 16 into the tapered shape, the shrink tube 18 is first contracted, and, then as shown in Figure 20, the distal region of the resin tube is converted into a tapered shape 21 as it is brought into contact with a heated mold 20 shown in Figure 19 having a desirable tapered shape 201 on the internal surface.

The shrink tube 18 is then separated as shown in Figure 21, and the internal layer tube 3, the reinforcing material layer 5, and the external layer tube 16 in the distal and proximal terminals of the catheter are cut or adjusted as needed. These processings are performed in the process C.

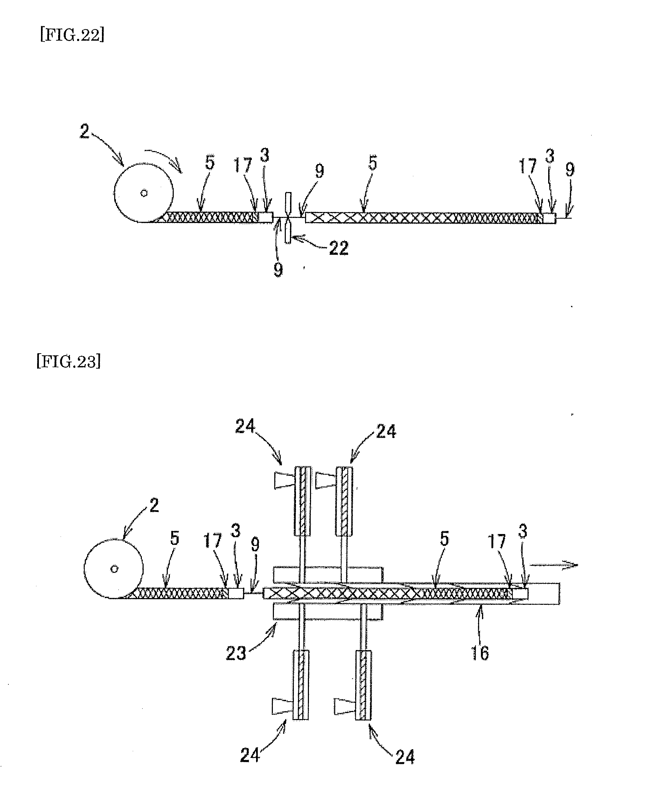

The process D is a step of connecting the catheters cut in the process B into a continuous member, while the metal core wires 9 are welded. Welding is performed, for example, in a spot welding machine 22 as shown in Figure 22, wherein the metal core wires are butt-welded, and the product is wound again around the reel 2. That is the processing in the process D.

The process E is a step of coating an external layer tube 16 continuously on the long-connected catheter by switched extrusion, and the internal layer tube 3, the reinforcing material layer 5, and the external layer tube 16 are integrated, while the external layer tube 16 is formed by coating extrusion in such a manner that the Shore D hardness changes in multi steps, or while the external layer tube 16 is formed by coating extrusion in such a manner that the Shore D hardness decreases gradually in the direction from the proximal region to the distal region when the Shore D hardness changes stepwise.

Then, when resins having multi-phase, for example, four-phase Shore D hardnesses are to be coated, as shown in Figure 23 the external layer tube 16 is formed by feeding the four resins from four extruders 24 that are connected to one extrusion mold 23, and adjusting the flow thereof to make the catheter have a desirable external diameter by operation and termination of the four extruders. Although not shown in the Figure, the external layer tube 16 may be formed by continuously extruding the resins different in Shore D hardness by four extruders connected to a mold having a valve mechanism, one by one into the extrusion channel by switching operation and termination of the extruders. Although not shown in the Figure, when an X-ray-radiopaque metal wire marker or an X-ray-radiopaque metal sheet marker is not used, a marker may be formed in the most distal region of the external layer tube 16 with a resin containing a kneaded X-ray-radiopaque metal powder.

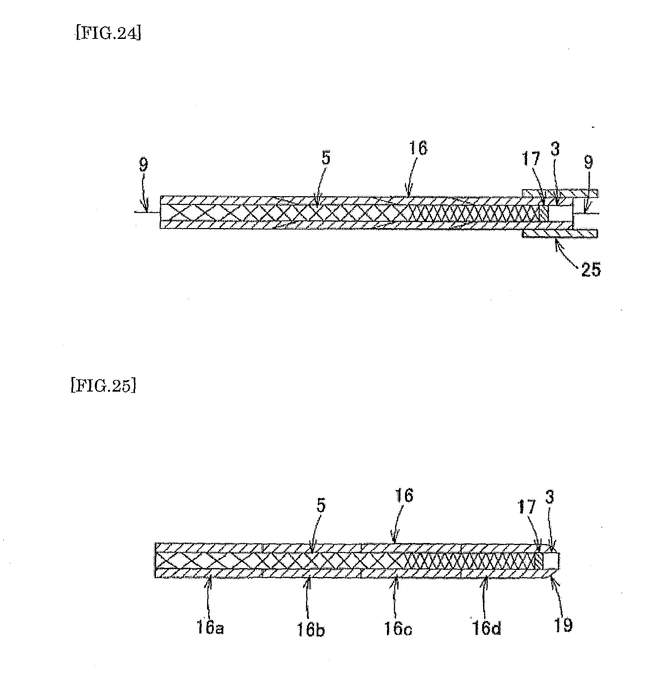

Then, the catheter is cut one by one; the terminal of the internal layer tube 3 or the external layer tube 16 in the distal region is adjusted; and a shrink tube 25 that shrinks in its diameter under heat is placed only at the distal end, as shown in Figure 24. The material for the shrink tube 25 is preferably polytetrafluoroethylene, a perfluoroethylene-propene copolymer, or the like.
Similarly to the process C above, the internal layer tube 3, the reinforcing material layer 5, and the external layer tube 16 are integrated in the following step, while the shrink tube 25 is heated to a tube-contracting temperature by a heater or application of high-frequency electromagnetic wave. The integration means that the internal layer tube 3, the reinforcing material layer 5, and the external layer tube 16 are bound to each other, while the mutual movement is restricted. Then as shown in Figure 18 the distal region of resin tube of the external layer tube 16 is converted into the rounded shape 19 by contraction of the shrink tube 25. In forming the distal region of resin tube of the external layer tube 16 in the tapered shape, as shown in Figure 20, the shrink tube 25 is first contracted and converted into the tapered shape 21, as it is brought into contact with the heated mold 20 in a way similarly to Figure 19. The shrink tube 25 is removed after the conversion.
That is the processing in the process E.

Subsequently in the process F, although not shown in the Figure, the catheter tube surface is preferably covered with a hydrophilic (or water-soluble) polymer substance, and thus, the composite is coated for improvement in hydrophilicity. In this way, it is possible to reduce the friction coefficient of the catheter, making it more lubricant, and to increase the lubricity of the catheter tube further even when the outermost surface of the catheter tube becomes in contact with blood, physiological saline, or the like, and consequently, to improve insertion efficiency, compatibility, kink resistance, and stability further. Examples of the hydrophilic polymer substances include the following natural or synthetic polymer substances and the derivatives thereof. Particularly favorable are cellulosic polymer substances (such as hydroxypropylcellulose), polyethylene oxide-based polymer substances (such as polyethylene glycol), maleic anhydride-based polymer substances (maleic anhydride copolymers such as methylvinylether-maleic anhydride copolymer), acrylamide-based polymer substances (such as polyacrylamide), and water-soluble nylons, because these materials give a low friction coefficient consistently.

Further as shown in Figure 25, the metal core wire is withdrawn, and the internal layer tube 3, the reinforcing material layer 5, and the external layer tube 16 at the proximal end are cleaved by means of a disk-shaped diamond cutter revolving at high speed, converting the proximal end cross section into a single plane and thus giving a catheter tube.

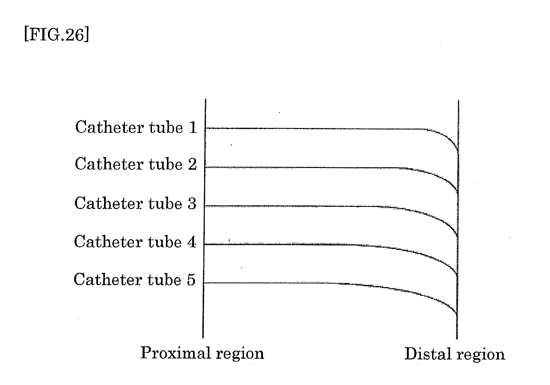

It is possible to make the rigidity/flexible inclination and the rigidity/flexible balance of the catheter tube suitable for various access routes more freely, by properly setting the braid pick distance, the length of the constant pick distance region, the order and the lengths of the resin tubes different in Shore D hardness. The rigidity/flexible balance is an indicator of the difference of the position of the high-flexibility region in the distal region or of the position of change in bending strength, as shown in Figure 26. In Figure 26, the catheter in the straight region is higher in rigidity than that in the distal region, and yet retains a favorable flexibility as well. Proper adjustment and selection of the rigidity/flexible balance gives various advantages, and, for example, a catheter tube similar to the tube 1 shown in Figure 26 transmits the condition in distal region directly at high sensitivity and transmits torque efficiently, while a catheter tube similar to the tube 5 allows easier insertion into a deeper affected area via a complicated route and transmits the intention of surgeon's operational method to the affected part more effectively.

When the internal layer tube 3 is formed with a fluorine resin such as polytetrafluoroethylene, the internal tube surface may be hydrophilized to a suitable degree by electrical means such as plasma discharge treatment.
In addition, although not shown in the Figure, it is possible to obtain a medical catheter tube in the most desirable shape by connecting a hub in a suitable shape to the proximal end.
The medical catheter may be used as it is as described above or as it is bent as needed while part of the medical catheter tube is heated by a heater or with steam.

## Claims

1. A medical catheter tube having a proximal region, a distal region and a most distal region from the proximal end, comprising: an internal layer tube of a resin tube; a reinforcing material layer of a wire braided on the internal layer tube; a marker formed by winding an X-ray-radiopaque metal part around the internal layer tube in the distal region; and an external layer tube of resin tubes covering the reinforcing material layer and the marker,
wherein:
the internal layer tube, the reinforcing material layer, the marker and the external layer tube are integrated;
the wire for the reinforcing material layer includes a synthetic resin wire and/or a metal wire;
the reinforcing material layer is formed only in the proximal region; the marker is flexible to bending deformation; and
the flexural rigidity of the external layer tube decreases stepwise or continuously in the direction from the proximal region to the distal region.

2. The catheter tube according to Claim 1,
wherein:
said resin tube is lubricating and flexible;
the wire for the reinforcing material layer provides the catheter tube with kink resistance, pressure resistance, torque-transmitting efficiency, insertion efficiency, and others; and
said external layer tube is flexible.

3. The medical catheter tube according to Claim 1 or 2,
wherein the marker is an X-ray-radiopaque metal wire coil wound around said internal layer tube, a square X-ray-radiopaque metal sheet having slits cut from both sides wound around said internal layer tube, or a tube of a resin kneaded with a X-ray-radiopaque metal powder.

4. The medical catheter tube according to any one of Claims 1 to 3,
wherein said wire for the reinforcing material layer is a synthetic fiber having a thermoplastic liquid crystal polymer as its internal core and a flexible polymer as its sheath.

5. The medical catheter tube according to any one of Claims 1 to 4,
wherein the pick distance of the braid for said reinforcing material layer changes continuously or stepwise in the direction from the proximal region to the distal region.

6. The medical catheter tube according to any one of Claims 1 to 5,
wherein said external layer tube has multiple segments, which are aligned in such a manner that the Shore D hardness of the resins for the segments decreases stepwise in the direction from the proximal region to the distal region.

7. The medical catheter tube according to any one of Claims 1 to 6,
wherein the external diameter of said external layer tube is altered and the catheter tube is formed in the rounded or tapered shape in the most distal region.

8. The medical catheter tube according to any one of Claims 1 to 7,
wherein said external layer tube is coated hydrophilically.

9. A process of producing the medical catheter tube according to any one of Claims 1 to 8, comprising forming a reinforcing material layer on the external surface of an internal layer tube by braiding, forming an X-ray-radiopaque marker flexible to bending deformation to the distal side of the reinforcing material layer, and coating an external layer tube,
wherein the X-ray-radiopaque marker is formed by winding an X-ray-radiopaque metal wire in the coil shape or by placing a square X-ray-radiopaque metal sheet having slits from the both sides around the internal layer tube to the distal side of the reinforcing material layer or by using a resin kneaded with a X-ray-radiopaque metal powder and thus the catheter tube has a flexible distal region.

10. A process of producing the medical catheter tube according to any one of Claims 1 to 8, comprising forming a reinforcing material layer on the external surface of an internal layer tube by braiding, forming an X-ray-radiopaque marker flexible to bending deformation to the distal side of the reinforcing material layer, and coating an external layer tube,
wherein the reinforcing material layer is formed by braiding a wire supplied from a wire supplying unit on the external surface of the internal layer tube in such a manner that the braid pick distance changes continuously or stepwise by changing the relative traveling speeds of the internal layer tube and the wire fed from the supplying unit.

11. A process of producing the medical catheter tube according to any one of Claims 1 to 8, comprising forming a reinforcing material layer on the external surface of an internal layer tube by braiding, forming an X-ray-radiopaque marker flexible to bending deformation to the distal side of the reinforcing material layer; and coating an external layer tube,
wherein:
one or more resin tubes different in Shore D hardness are formed as the external layer tube; and
the rigidity/flexible balance of the catheter tube can be altered in various ways by aligning the resin tubes for external layer tube in such a manner that the Shore D hardnesses thereof decreases in the direction from the proximal region to the distal region and also in such a manner that the braid pick distance of the reinforcing material layer changes continuously or stepwise when multiple resin tubes different in Shore D hardness are formed.

12. A process of producing the medical catheter tube according to any one of Claims 1 to 8, comprising forming a reinforcing material layer on the external surface of an internal layer tube by braiding, forming an X-ray-radiopaque marker flexible to bending deformation to the distal side of the reinforcing material layer, and coating an external layer tube, further comprising forming one or more resin tubes different in Shore D hardness as the external layer tube, aligning the resin tubes for external layer tube in such a manner that the Shore D hardnesses thereof decreases stepwise in the direction from the proximal region to the distal region for making the stepwise change of the resin tubes different in Shore D hardness, covering the entire composite with a shrink tube, the internal layer tube, the reinforcing material layer, the X-ray-radiopaque marker, and the external layer tube by heating the composite, converting the most distal region thereof into the rounded or tapered shape, and removing the shrink tube after cooling.

13. A process of producing the medical catheter tube according to any one of Claims 1 to 8, comprising forming a reinforcing material layer on the external surface of an internal layer tube by braiding, forming an X-ray-radiopaque marker flexible to bending deformation to the distal side of the reinforcing material layer, and coating an external layer tube, further comprising forming one or more resin tubes different in Shore D hardness as the external layer tube, by coating extrusion, on a structure having a reinforcing material layer formed by coating extrusion molding on the external surface of the internal layer tube in such a manner that the Shore D hardness changes stepwise, forming the resin tubes for external layer tube by coating extrusion in such a manner that the Shore D hardnesses thereof decreases stepwise in the direction from the proximal region to the distal region for making the stepwise change of the resin tubes different in Shore D hardness, integrating the internal layer tube, the reinforcing material layer, the X-ray-radiopaque marker, and the external layer tube, and converting the most distal region into the rounded or tapered shape.
